# EUROPEAN PATENT SPECIFICATION

(11) **EP 1 005 379 B1**
(45) Date of publication and mention of the grant of the patent: **18.02.2004**
(21) Application number: 98938777.4
(22) Date of filing: 19.08.1998
(51) Int. Cl.: A61L 27/58, A61L 27/44

(54) **BIODEGRADABLE COMPOSITES**
BIOLOGISCH ABBAUBARE VERBUNDWERKSTOFF
COMPOSITES BIODEGRADABLES

(30) Priority: 19.08.1997 GB 9717433
(43) Date of publication of application: 07.06.2000
(73) Proprietor: THE UNIVERSITY OF NOTTINGHAM, Nottingham NG7 2RD (GB)
(72) Inventor: CORDEN, Thomas Joseph, University Park, Nottingham NG7 2RD (GB); DOWNES, Sandra School of Biomedical Sciences, Nottingham NG7 2RD (GB); FISHER, Sheila, Eunice (Queen's Medical Centre), Nottingham NG1 5DW (GB); JONES, Ivor, Arthur, University Park, Nottingham NG7 2RD (GB); RUDD, Christopher, Douglas, University Park, Nottingham NG7 2RD (GB)
(74) Representative: Lawrence, John
(86) International application number: PCT/GB1998/002399
(87) International publication number: WO 1999/011297

(56) References cited:
- EP-A- 0 192 068
- WO-A-95/07509
- US-A- 5 108 755
- CHEMICAL ABSTRACTS, vol. 125, no. 2, 8 July 1996 Columbus, Ohio, US; abstract no. 12759, XP002097606 cited in the application & C.D. RUDD ET AL.. EDITORS: "Liquid molding technology: a guide to RTM, SRIM and related composites processing techniques " 1996 , WOODHEAD , CAMBRIDGE UK

## Description

The present invention relates to a biocompatable, biodegradable composite, production and/or preparation thereof, for use in surgical procedures such as surgical implantation and bone fixation, resurfacing and augmentation procedures.

Despite numerous examples of the use of synthetic, permanent implant materials such as acrylic polymer, silicone elastomer, ceramic polymer composites, polymethylmethacrylate, polyethylene and porous PTFE-carbon fibre composite, the reconstruction of traumatic, developmental and surgical osseous defects is largely dependent upon an adequate supply of autogenous (host) or allogeneic (donor) bone. Bone autograft is widely considered the best implant material for repairing bone defects, simply because of the reduced likelihood of rejection and concomitant immunological problems. However, the amount of autogenous bone available for transplantation is limited since it has to be taken from a part of the host's own body. Furthermore, the harvesting operation itself carries with it the risk of post-operative complications, in some instances this risk is of a greater magnitude than the primary procedure itself especially if the individual has recently suffered severe trauma.

Common donor sites include bone material of the iliac crest, tibia, fibula and greater trochanter. Bone itself has at least two distinct types, and selection of bone type is dependent upon its intended implant site and function. Cortical bone (that is the outer layers) is selected for its strength and mechanical support, whilst cancellous bone (that is the more spongy form) autografts are used to promote lattice formation and rapid bone regeneration. Autografts of either and/or both types have been used extensively and successfully used in oral and maxillofacial surgery for restoration of the periodontium and correction of mandibular and maxillary defects.

Rapid and extensive vascularisation of the graft is important for survival of the bone graft and supply of appropriate nutrients and the like to cells. However, problems have been encountered using autograft bone due to shrinkage of the graft material itself and partial and variable resorption of the osteons and hence restricted regenerative capacity of new bone. It is of note that whilst allografted (where bone is transplanted between two individuals - often from cadaveric donors with specimens being kept in bone banks) bone avoids the potential risks of a harvesting operation it offers a potentially unlimited material in banked form. Nevertheless, banking of bone is a complex procedure involving extensive, time consuming and expensive procedures:- donor selection, screening, procurement and storage. Moreover, the possible transmission of diseases such as Creutzfeldt-Jacob or AIDS raises significant and potentially lethal problems with its use. Several years may be required for reabsorption and replacement of allograft by new bone and the antigenic activity of non-host bone is a serious disadvantage compared with autografts. As a consequence, the search for suitable alternatives to host and donor bones has intensified.

It is known to provide bioceramics of calcium phosphate, typically these are in the form of biodegradable tricalcium phosphate and hydroxyapatite products.

Furthermore such bioceramics display advantages of biocompatability, osteoconductive capability and chemical similarity to mineralised bone matrix which results in direct bonding to bone. Consequently they satisfy most of the essential criteria for successful bone grafting. However, significantly, bioceramics do not appear to induce pronounced osteogenesis. Furthermore the inherent hardness of bioceramics render them difficult to shape thus bioceramics have limited use within an animal skeleton as the material cannot be readily shaped to the defect. Moreover, the rigidity is also a disadvantage as the healing progresses because the rigid plate causes stress shielding around the fracture site and as a consequence the bone is not subject to the normal force induced remodelling at the site of fracture closure. This can be a serious problem if the fracture plate is removed, with the underlying bone being unable to handle the forces acting upon it and a refracture may result Additionally and disadvantageously bioceramics also remain in the repair site for extended periods, typically more than one year.

Additionally it is known to use mixtures of collagen/ceramic/marrow, with the aim of replicating the organic matrix/mineral phase/osteogenic cell structure of bone. However, such mixtures have a limited capability in that they are useful only in fracture repair partially due to the paste-like quality of the mixture and hence difficulty in accurate and permanent placement and retention of the material at the site requiring repair.

It is recognised that a synthetic, re-absorbable polymeric implant could overcome many of the problems associated with the prior art not least the supply difficulties, long reabsorption time of the implant and bone union times vis-á-vis the implant; moreover the novel implant would immediately provide an advantage to current practices.

Notably, there is currently no successful biocompatible and/or biodegradable material for reconstructive surgery of bone in the face and skull and associated areas of disfigurement. Surgery to the face and skull following trauma, injuries, correction of congenital or acquired deformities and ablation of tumours can leave areas of bone discontinuity and/or distortion. Untreated bony defects can cause marked functional disability and disfigurement, furthermore disfigurement can be psychologically damaging and cause a great deal of personal and/or familial anxiety. Reconstructive surgery is an extremely important area of modern surgery and advanced techniques can lead to remarkable results. The current surgical procedures involve the replacement of bone structures with means as herein before described in addition to metal plates such as titanium alloys, cobalt-chromium alloys, and sculptured polyethylene for replacement of tissue sections and/or bony defects. The use of metal plates however has become increasingly less popular due to interference with medical imaging, consequently an investigator is unable to analyse the state of tissue (eg brain) or the like covered by said plate. Effectively the plate prevents imaging of tissue behind the plate. Moreover metallic fracture plates are not ideal for maxillofacial skull or long bone reconstruction. The delicate nature of facial bone requires miniature fixation screws, causing associated problems of obtaining a reliable joint. The complex facial geometry necessitates special plates and techniques, particularly in areas such as the orbital floor. Furthermore metallic plates can in some cases be visual and palpable below the skin and in many cases these plates have to be removed requiring a second operation with all the associated risks and costs. The surgical approach required to retrieve plates can be a complex and lengthy procedure. In other bones, plates are routinely removed, an inevitable cause of morbidity.

All biomaterials currently commercially available for cranio-facial and maxillofacial reconstructive surgery have significant problems including Proplast (polyethylene), Silastic (silicone), hydroxyapatite and bioactive glass granules. Problems with these and other materials include migration of the implant, formation of cold abscesses, lack of colour compatibility, lack of dimensional stability and difficulty in shaping of the material to "fit" the defect. Bone from allograft and autograft sources are also difficult to sculpture to a specific implant site and furthermore sculpting of bone can destroy/damage the living cells.

The ideal biomaterial for maxillofacial and other types of bony/cartilaginous reconstruction will have numerous properties. It should be biocompatible, capable of facilitating revascularisation and cell growth providing a framework to guide the new bone development. The material needs to be sterile, malleable, storable and affordable. It could also act as a carrier mechanism for osteogenic proteins. A high initial stiffness will allow primary union followed by gradual resorption and reduction in stiffness corresponding with the healing bone's ability to serve in a load bearing capacity. Ideally the material should be easily processed into complex shaped components. With the use of CT patient scan data this creates the possibility of producing accurate tailored implants for elaborate reconstructive surgery.

The ability to vary the degradation rate of biocompatible relatively short length - polyesters such as polylactide and polyglycolide by copolymerization, and to control molecular weight, crystallinity and morphology has made these two materials natural candidates for bone repair and are the most promising materials in the development phase. However they remain far from ideal.

Poly-ε-caprolactone (PCL) is a relatively long -polyester hydrocarbon chain thermoplastic (Tm = 60°C) having a low elastic modulus which mitigates against its use in bone implants without some structural reinforcement. The characteristics of PCL increases its relative permeability with respect to other -polyesters and thus PCL has been exploited as a vehicle for diffusion controlled delivery of low molecular weight (MW 400) drugs and has been used in the area of contraceptive therapeutics.

US 4,655,777 and US 5,108,755 disclose composites comprising PCL matrix reinforced with certain biodegradable fibres for improved retention of yield strength and modulus with time under degrading conditions. In US 5,108,755 is disclosed a need for composites providing prompt clearance from the system without premature compromising degradation. In US 4,655,777 is disclosed matrix reinforced with biodegradable long, continuous fibres for increased strength. The composites are prepared using conventional processing routes.

Nevertheless there is a need for a method to provide shaped composites suited for the above mentioned applications in the form of pins, plates or custom shaped implants, for which the existing processes are lacking in convenience and versatility. There is moreover a need for shaped composites having improved performance as bone repair materials.

It is therefore a first object of the invention to provide a biocompatible composite for use in transplant surgery, bony resurfacing or the fixation of fractures and/or tissue scaffolding.

It is yet a further object of the invention to provide a biocompatible composite for use in cranio-facial or maxillo-facial surgery, some applications of orthopaedic surgery such as replacement of bone/cartilage/meniscus.

It is yet a further object of the invention to provide a biocompatible composite which may be moulded to any size or shape that it is desired to implant/reconstruct.

It is yet a further object of the invention to provide a biocompatible composite which is fully biodegradable.

We have now unexpectedly found that by use of a specific process for processing composites for the presently envisaged applications in shaped form, excellent results in terms of processing convenience and product quality are obtained. We have moreover found that degradation may be predetermined in manner to provide custom composites adapted for implant/reconstructive surgery with excellent recovery time.

We have also found that the process and products are suited for new applications further enhancing the versatility of the technology.

According to the invention, a biodegradable fibre reinforced shaped composite suitable for use as a medical implant comprises matrix and fibres wherein the matrix and fibres display differential rates of biodegradation as a function of the nature of material or molecular weight thereof characterised in that the shaped composite comprises a shaped fibre preform of predetermined fibre distribution, orientation and/or fraction and the matrix is selected from polymers and co-polymers of aliphatic polyesters, preferably poly-ε-caprolactone, such that, in use, the matrix and/or fibre biodegrade via an intermediate comprising residual porous matrix or residual fibre form respectively providing voids suitable for primary growth of cells or providing a residual scaffold for attachment and growth of cells.

Use as medical implant may include any known use for example selected from cranial, maxillofacial and orthopaedic surgery for the purpose of fixation, augmentation and filling in of defects.

The novel composites are of any desired 3 dimensional geometry which may be complex, having chemical and mechanical properties comparable to those of composites obtained using conventional bulk polymerisation processes. Preferably the composites are shaped in the form of pins, plates, meshes, screws, rivets and/or custom shaped implants to fit the contour of the area to be constructed and to secure the device, optionally made to a range of sizes for more general use or the manufacture of plates and fixation devices to support bone during healing.

For example a custom implant for augmentation of filling of defects may comprise associated devices for fixation. Restoration of bone or other biological tissues such as cartilage, may be envisaged.

A process for making such a composite may comprise providing the said shaped fibre preform comprising a presentation of reinforcing fibres in a regular, irregular or profiled fibre distribution in a tool or mould;
injecting into said preform within said tool or mould a composition comprising monomers or co monomers and/or oligomers and/or resin of the said polymer matrix in such a manner as to retain said distribution, orientation and/or fraction, of fibres and composite shape; and
polymerising or part polymerising the composition in the mould or tool.

A shaped fibre preform as hereinbefore defined may be any presentation of fibres in a suitable tool, mould or the like adapted for impregnation with polymer or polymer precursors to provide a composite having irregular shape. The shaped fibre preform preferably enables a predetermined regular, irregular and/or otherwise profiled fibre distribution.

Fibres may be any natural or synthetic loose, aligned, knitted or woven material or fabric having length and direction selected for desired mechanical properties. Short fibres which are up to 10² times greater in length than diameter may be employed where only moderate load bearing strength is required, or long continuous fibres which are 10² - 10⁴ times greater in length than diameter may be employed where high load bearing strength is required.

It has been found that the composition processed *in situ* provides accuracy, ease and convenience of handling and shaping to provide a shaped composite, without compromising the excellent properties in terms of modulus and strength, provided by the fibre reinforcement and matrix. The composition may moreover be selected to provide polymer matrix of desired molecular weight, adapted for the required degradation profile, irrespective of concerns over ease of impregnation of fibres, example with use of high molecular weight, high viscosity polymers

The composition of the invention are found to be ideally suited for the intended uses by virtue of their versatility to provide high quality high strength implants adapted in novel manner for biocompatibility and cell growth by controlled or differential degradation.

Fibre reinforcement is selected from a plurality of suitable, synthetic and/or natural fibres selected from ceramics such as beta-tricalcium phosphate and phosphate free calcium aluminium (Ca-Al), bioglasses such as the glass form of calcium phosphate, calcium metaphosphate (CMP) and calcium sodium metaphosphate (CSM), mixtures of silica, sodium oxide, calcium oxide and phosphorus pentoxide.

The composite of the invention is preferably obtained by polymerisation using a modified resin transfer moulding technique. Resin transfer moulding (RTM) is a composite manufacturing technique normally used with thermosetting resins ⁽¹⁾. A reactive liquid resin is injected into a tool cavity containing a dry fibre preform. The resin wets out and infiltrates into the fibre bundles and upon curing produces a composite thermoset material.

RTM is preferably adapted as a manufacturing technique for biocompatible biodegradable polymer matrices such as PCL as hereinbefore defined. The novel process allows the production of complex shaped biobsorbable composite materials. Preferably fibre fractions and directions are controlled. The low pressure process requires only economic lightweight tooling and injection equipment allowing us to produce thermoplastic components without the normal expense of conventional injection moulding tooling and machinery.

A mould for preparing a preform as hereinbefore defined may be constructed of any desired natural or synthetic material having temperature resistance in excess of the processing temperature to be employed in processing the composite. Suitable materials for constructing the mould include steel, aluminium and the like which may be coated with release agents as known in the art, for example wax, poly vinyl alcohol, silicone based agents and the like, or is constructed entirely from materials have release properties, for example is machined from PTFE.

The mould may be of any desired construction suitable for injection of resin into a preformed fibre bundle or the like. For example the mould may comprise a portion having a machined cavity and a further portion having inlet and outlet ports for introduction of resin and release of volatile and bleed excess resins.

The composite may be obtained by polymerisation by suitable means, preferably by heating or by addition of an initiator or catalyst which may be present in or added to the composition *in situ.*

A composite comprising PCL for example is suitably obtained by cationic polymerisation for example using an organometallic catalyst such as organozinc, preferably diethylzinc. The catalyst may be adapted to coordinate to a reactive group such as carbonyl on caprolactone resulting in cleavage of a bond and cation formation which can then add to a further caprolactone resulting in the growth of the polymer chain. The method results in well defined polymers with high molecular weight and narrow polydispersities (<2).The lack of branching by this method also gives higher crystallinity and higher Tm, and therefore superior material properties, which are thought to be more appropriate in the biodegradation process.

It is a particular advantage that the process which can be carried out at low pressure and using lightweight tooling, as described above, may be adapted for preparing shaped composites non-industrially with use of a small scale or portable moulding unit for immediate use, dispensing with the need to commission in advance from an industrial manufacturing source. This has clear benefits in terms of customising shaped composites to be produced as a one-off product.

Surprisingly, we have found that PCL is highly biocompatible with osteoblasts. Moreover, unlike most biodegradable polymers, which tend to degrade via bulk hydrolysis to monomer constituents with a sudden breakdown of the material resulting in large amounts of degradation products lowering the surrounding pH and producing inflammatory/foreign body responses, PCL bioerodes at the surface, a phenomenon which advantageously allows for rapid replication of bone cells and remodelling of bone during biodegradation. Typically osteoblasts infiltrate into the matrix and allow the bone to form around the fibres, thus providing good implant bonding and maintaining biological and mechanical integrity. Furthermore the use of PCL as a matrix in a long fibre composite material should give significant scope for the tailoring of mechanical and degradation properties by varying the matrix molecular weight and the fibre orientation and fraction.

The invention of the application also concerns the serendipitous finding that a PCL matrix, reinforced with long fibres, biodegrades at a slower rate and differentially so that during bone remodelling, osteoblasts migrate into the PCL matrix and allow the bone matrix to form around the fibre, thus maintaining mechanical and biological integrity. Consequently the observed preferential biodegradation of the matrix material allows osteoblasts to infiltrate and differentiate into osteocytes and to grow around the long fibres, the fibres themselves biodegrade only after the bone has substantially formed and regrown. Therefore, the development of a totally bioabsorbable long fibre composite material allows a two stage degradation to occur with a differential rate of degradation between the components such that one degrades first leaving a void or scaffold structure of the other which would be absorbed at a later stage.

The composite may be suitably selected for primary growth of cells selected from bone, cartilage, or a live vascular structure within the partially degraded composite, adapted for further growth of remaining cells types for total integration as a functioning live system.

The differential degradation composites of the invention provide the continuity of mechanical integrity and the intended preferential degradation mechanism in which the matrix or fibres degrade only after bone or vascular formation respectively within the composite matrix.

Degradation rate of a material may be determined by means known in the art and selection of respective materials having a desired differential may be made. It is convenient to classify materials according to slow, medium and fast degradation rates whereby selection of material having the appropriate rate may be made together with any other desired physical, mechanical and chemical properties for the intended use.

Either matrix or fibre may be adapted for primary degradation, with the other being adapted for secondary degradation. Preferably matrix is selected for primary degradation when it is desired to implant for reconstruction of bone or cartilage or the like. Preferably fibre is selected for primary degradation when it is desired to implant for reconstruction of soft tissue, muscle or the like.

The nature of fibres may also be selected to provide a desired void or residual structure specifically adapted to promote a desired vascular/muscle or bone/cartilage structure. For example a parallel aligned fibre preform of continuous long fibres will create a different void or residual structure to that of a felt or knitted or woven mat of short non-aligned fibres, which may be specifically selected to mimic a living structure or to provide a scaffold on which a living structure can most efficiently establish itself.

A shaped composite as hereinbefore defined may be coated with or associated with or have embedded therein or be impregnated with an appropriate therapeutic agent. Preferably the therapeutic agent is an antibiotic and/or a growth promoter and/or a vitamin supplement which aids implantation, growth and take of said curable composition.

A shaped composite as hereinbefore defined may be coated with or associated with or have embedded therein or be impregnated with a selected population of host and/or compatible donor cells. Preferably the cells are bone derived and/or cartilage derived and/or collagen derived. The selection of said cells is dependent on the intended implant site and inclusion of said cells is intended to aid implantation, growth and take of said curable composition at the site of implantation.

Furthermore, we have inventively discovered a means for matching the implant geometry exactly to the patient, by use of medical imaging and liquid moulding of the composite to a dimensionally accurate surgical feature construct.

It will be appreciated by those skilled in the art of surgical reconstruction that the use of the composite of the invention is not intended to be limited to use in bony areas of the face and skull but is intended to be used on any part of the body of an animal or human that has ossification and/or cartilage and/or meniscus that requires surgical reconstruction and so the examples referred to herein are not intended to limit the scope of the application. Additionally it will be appreciated that reconstructive surgery is intended to include cosmetic surgery and surgery for aesthetic purposes.

The composite may moreover be impregnated with cells as hereinbefore defined.

In a further embodiment that the composite may be used as a template for in vivo tissue production using bioengineering techniques as known in the art. In this embodiment the impregnation may be with cells as hereinbefore defined, inductive proteins, therapeutic substances and the like, and the composite is then adapted for introduction into a living host, such as the human or animal body or a part thereof, and subsequently harvesting the composite in partial or substantially impregnated and/or degraded state and reimplanting in a locus for reconstructive surgery.

Implant may be into muscle for attachment and growth of living cells, with subsequent harvesting at the time of definitive surgery, for example in cranial, maxillofacial, orthopaedic and the like surgery as hereinbefore defined to provide bone, cartilage and the like.

In a production process as described above, the mould or tool may comprise a 3-dimensional template of a 3-dimensional image of a selected feature or area of a patient for implant. In this case, the mould or tool may be provided by
(i) medical imaging of a selected feature or area of a patient complementary to or symmetrical with a feature or area to be replaced and/or restructured to obtain data comprising a plurality of coordinates defining a three dimensional image;
(ii) passing data collected from medical imaging to a translating system which interprets said data and generates information for transferring said data to a rapid prototyping system suitable for generating a mould or tool;
and wherein fibre comprises long continuous fibres which are 10² -10⁴ times greater in length than diameter providing directional reinforcement.

Preferably, the process includes liquid moulding a product to a specified size and shape, by introducing a suitable amount of matrix resin as hereinbefore defined for example; caprolactone and/or biocompatible derivatives and/or analogues thereof; and fibres as hereinbefore defined, for example long, or directional continuous, fibre-reinforcement; and catalyst and/or initiator into a mould under conditions that favour in-situ polymerisation of matrix; curing the composite by appropriate means; removing the mould from a cured shaped product; and optionally preparing the shaped product for introduction into a recipient by appropriate means.

In this work, catalysed caprolactone monomer is injected into a tool cavity to produce test plagues of PCL. Specimens with different molecular weights have been produced and the physical and biocompatability characteristics of this insitu polymerised material compared to commercially available PCL. The effect of gamma sterilisation has also been investigated as this is the most likely sterilisation procedure to be used for such implants. A cell culture system with bone cells derived from craniofacial bone cells (CFC) has been used to assess the biocompatability of the PCL material. Finally, totally bioabsorbable long fibre reinforced composite materials have been manufactured using this in-situ polymerisation technique using both knitted and woven Vicryl meshes produced from a polylactic acid/polyglycolic acid (PLA/PGA) copolymer.

The invention will now be described, by way of example only, with reference to the following figures, wherein:-
(i) Figure 1 represents a block schematic representation of the process of the invention.
(ii) Figure 2 represents a front cross-sectional view of the apparatus employed in in-situ polymerisation of polycaprolactone.

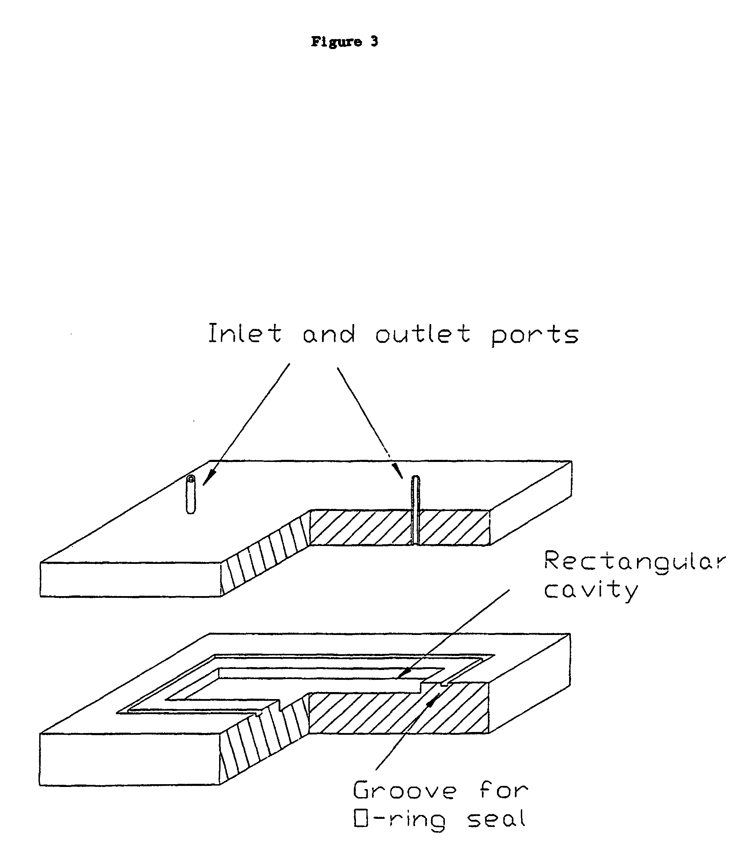

Figure 3 represents a perspective, partial cross-sectional view of a machined PTFE rectangular cavity mould.
Figure 4 - GPC curves showing molecular weight distribution;
   unsterilised PCL 75; b) gamma sterilised PCL 75.
(v) Figure 5 - Tensile modulus Vs molecular weight for unsterilised and gamma sterilised PCL □: unsterilised *in-situ* polymerised PCL, O: gamma sterilised *in-situ* polymerised PCL, X unsterilised CAPA 650 (measured value), • : gamma sterilised CAPA 650 (measured value), ■ : unsterilised CAPA 650 (Solvay value).
Figure 6 - H¹ NMR spectra for PCL 50.
Figure 7 - H¹ NMR spectra for CAPA 650.
viii) Figure 8 - Reflection IR spectra; a) CAPA 650 b) PCL 50
ix) Figure 9 shows a cross-sectional view of knitted Vicryl mesh/PCL composite showing the knitted mesh to be fully integrated with the PCL matrix material. Note also the twisted, knitted structure of the Vicryl mesh.
x) Figure 10 shows a cross-sectional woven Vicryl mesh/PCL composite showing the woven structure of the Vicryl mesh.
xi) Figure 11 shows individual Vicryl fibres fully wet out and encapsulated within the PCL matrix material.
Figure 12 shows an Alamar Blue assay of CFC on PCL of different molecular weights after 48 hours.

### MATERIALS AND METHODS

### Modelling Curable Composition

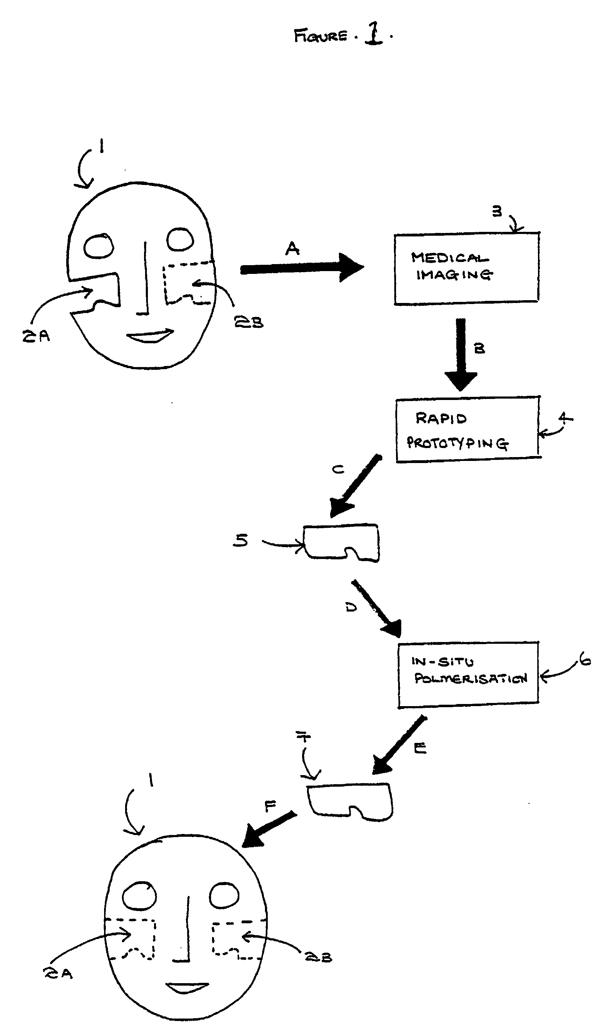

With reference to Figure 1, there is shown an individual's face (1) wherein area 2A represents a feature or area to be surgically treated. Area 2B represents a complementary feature or area, typically symmetrical with the feature or area to be treated. In order to match the implant geometry closely to the patient, medical imaging (3) such as CT and/or MRI and/or NMR ( or MRI) scanners are used to provide three dimensional data of a complementary feature or area. In the instance where a complementary feature or area does not exist, or is not suitable, data derived from a compatible or average image may be used in the working of the invention. Optionally the medical imaging data may be mirror imaged so as to provide an image of appropriate hand.

Medical imaging data is then processed along arrow B in a conventional manner so as to provide data in the correct form for rapid prototyping (4). Rapid prototyping is a means by which moulds for liquid moulding can be made, directly or indirectly, and it will be appreciated by those skilled in the art of providing an implant that this particular procedure is not intended to limit the scope of the application but merely to provide a means by which a preformed mould (5) may be produced. Following along process D, the closed mould (5) in which a preform of synthetic and/or natural fibres is placed along with an appropriate amount of caprolactone is then subjected to in-situ polymerisation (6). (This process will be described in greater detail in the following text.) Subsequent to polymerisation and curing of the polycaprolactone material the mould is removed along process E so as to provide a shaped product (7) which upon appropriate deflashing and preparation is implanted following process F into the appropriate position of an individual's face (1).

In this way it is apparent that the process of the invention requires multiple integrated steps, the exact nature of which is not intended to limit the scope of the application but merely provide examples of the ways and means of providing a shaped product for transplantation from the composite of the invention.

For more general use a series of sized moulds may be used to provide a range of preformed implants, plates, fixation devices and the like as hereinbefore defined.

### In-situ polymerisation of polycaprolactone

### Monomer Preparation

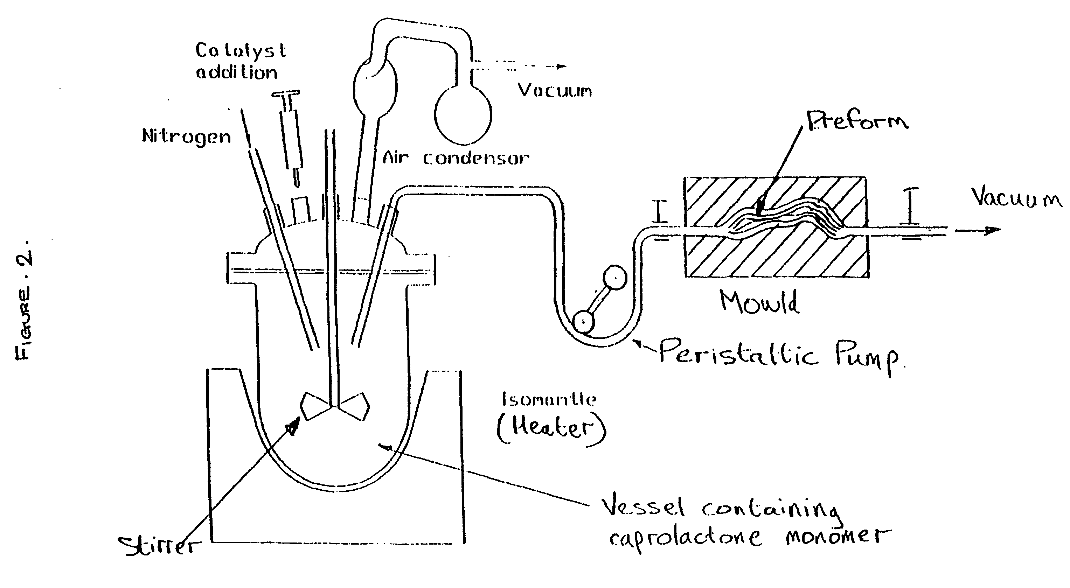

ε-Caprolactone monomer (Solvay Interox, Widnes, UK) was purified by distillation under reduced pressure over freshly powdered calcium hydride. The reaction apparatus is outlined in figure 2. Distilled caprolactone monomer dried over molecular sieves was charged into a 500 ml round bottom five-necked flask fitted with a Teflon blade stirrer, an inlet for dry nitrogen gas, a thermocouple probe, a rubber septum inlet and a outlet pipe. Attached in line with the outlet pipe was a machined PTFE rectangular cavity mould with a peripheral nitrile O-ring seal with the mould outlet attached to a vacuum pump. Initiator in the form of 1,4 butane-diol contained within low molecular weight (Mw 4000) powdered PCL (Capa 240, Solvay Interox) was added in the quantity required to give the desired molecular weight as detailed in table 1.

### Polymer and Composite Production

The mixture was heated to 80°C with an oil bath and stirred under nitrogen for 2 h. 500 ppm di-ethyl zinc ((C₂H₅)₂Zn) catalyst as a 15 wt. -% solution in toluene was added with a syringe via the septum inlet followed immediately by vigorous stirring for 30 s during which the mould cavity and reaction vessel were evacuated to 0.2 bar absolute in order to degas the monomer. The stirring was stopped and after a further 30s - 60 s degassing the pressure in the vessel was increased to ambient, the mould inlet tube pushed down into the monomer and the nitrogen pressure used to inject the catalysed monomer into the mould cavity. Upon filling the cavity the inlet and outlet pipes were clamped shut and the mould heated in an oven to 120°C for 18 h. Finally the mould was allowed to cool to room temperature and the polymerised PCL moulding removed from the mould. Two moulds were used, one with cavity dimensions 240 x 130 x 3 mm used for producing material for tensile and biocompatibility test specimens and a smaller one 80 x 30 x 3 mm used for producing the composite specimens. The fibre preforms consisted of 12 layers of either woven or knitted Vicryl mesh (polyglactin 910 from Ethicon, Edinburgh) cut to fit the mould cavity and vacuum dried over molecular sieves for 12 h at 120°C. The knitted material tends to deform at temperature so this was dried while clamped between aluminium plates.

### Comparative sample preparation

PCL (CAPA 650, Solvay Interox) was obtained in 3mm thick compression moulded sheets. This is a commercially available PCL with a nominal Mn of 50,000 and was used as a bench mark material to compare with the samples produced using our *in-situ* manufacturing technique.

Tensile test specimens were prepared by machining the PCL sheets into rectangular strip specimens 40 x 10 x 3 mm using a high speed fly cutter. Disc specimens for biocompatibility testing were produced using a 10 mm diameter circular punch. Both tensile and biocompatibility test specimens were sterilised with gamma radiation using an irradiation does of 27.8 kGy.

Having regard to the above the following procedure is a summary of the procedure used to polymerise caprolactone in-situ:-
(i) Construct the preform of dry synthetic and/or natural fibres to the required length and/or geometry, and place in mould.
(ii) Heat the mould to a suitable temperature below the melting/degradation point of the fibres and purge with dry nitrogen, or the like.
(iii) Distil the caprolactone monomer or oligomer at reduced pressure over a suitable anhydrous salt such as calcium hydride so as to remove impurities.
(iv) Heat the caprolactone monomer to a selected temperature under reduced pressure so as to remove any entrained air.
(v) In a vessel purged with nitrogen, add a stoichiometric quantity of an appropriate initiator such as (1,4-butanediol) and 50-250 ppm of a suitable catalyst such as (diethyl zinc in toluene) using syringes which have been dried and nitrogen-purged. Mix thoroughly.
(vi) Using a peristaltic pump and thoroughly-dried silicone tubing, pump the reaction mixture into an evacuated mould containing the preform of biodegradable fibres. When filled, seal entry and exit points and heat to 100° to 140° for an appropriate period until polymerisation has taken place (typical times appear to be 5 hours).

Deflash and tidy up the moulding prior to treatment and/or use for implantation.

### Measurements

Gel permeation chromatography (GPC Polymer Laboratories) was performed to determine the molecular weight distributions. Mixed D columns calibrated with polystyrene narrow standards (Polymer Laboratories PS-1) were used with 100 mg of polymer dissolved in 5mL of chloroform as the mobile phase.

Tensile modulus was measured with a Instron 1195 tensile testing machine using a clip-on electrical extensometer with a 10 mm gauge length a 5 kN load cell and a cross head speed of 1 mm/min.

Reflection infra-red spectroscopy was undertaken using a Perkin-Elmer system 2000 FT-IR spectrometer.

H¹ NMR of the sample and comparison recorded in CDCL₃, on a Bruker 300MHz FT-NMR using tetramethyl silane as the internal standard to assess similarity of the materials via their electronic structure.

Differential scanning calorimetry (DSC) was used to determine the melting temperature (Tm) and crystallinity of the PCL specimens. A Dupont Instruments 910 DSC calibrated with Indium was used with a starting temperature of -80°C and a heating rate of 10°C/min.

Biocompatibility Testing

### Cell Culture

Cranio facial osteoblast-like cells (CFC) were derived from bone fragments of skull from a 14 month old female. This method was based on that described by Robey and Termaine ⁽²⁾. Bone fragments were cut into small pieces, no more than 5mm in diameter, rinsed in sterile phosphate-buffered saline (PBS) to remove blood and debris, then plated out in 35mm diameter tissue culture plastic dishes (Falcon, Becton Dickinson Labware, Franklin Lakes, NJ, USA). Bone chips were cultured in complete Dulbecco's Modified Eagle Medium (DMEM) supplemented with 10% fetal bovine serum (FBS), 1% L-glutamine, 1% non-essential amino acids (NEAA), 2% Hepes buffer, 2% penicillin/streptomycin (all Gibco, Paisley, UK) 150µg/l L-ascorbic acid (Sigma, Poole, UK) and 1µg/ml Fungizone (Gibco) and incubated at 37°C in 5% CO₂ humidified atmosphere. Bone chip cultures were screened daily and culture medium changed every two days.

After several days, seams of bone cells formed around the edges of the bone chips and cells then began to attach to the tissue culture plastic and spread out. Within 2-3 weeks sufficient bone cells had grown out from the bone chips to be cultured alone. The bone chips were removed from culture and digested in 0.02% trypsin/0.03% collagenase in PBS incubated at 37°C for 20 minutes, rotated continually. The bone chips were discarded and the supernatant was centrifuged at 1200 rpm for 5 minutes to produce a cell pellet which was then resuspended in DMEM and centrifuged again to rinse off the trypsin/collagenase solution. The resulting cell pellet was resuspended and replated in 25 cm² tissue culture plastic flask (Falcon). Cells were grown to confluency and then passaged with 0.02% trypsin/01.M Herpes in PBS. Cells were characterised as osteoblast-like by morphological, ultrastructural and biochemical techniques, primarily by the expression of alkaline phosphatase, a marker of osteoblastic phenotype.

### Biocompatibility

Cells were seeded onto gamma-irradiated and non-irradiated polymer discs of different molecular weights. Two sets of polymers discs were used: 10 mm diameter discs were used for cell activity and morphology; and 8mm diameter discs for morphological assessment only. Tissue culture plastic or Thermanox® discs were used as an example of an optimum material and copper discs as an example of a material of poor biocompatibility. The non-irradiated polymers and the copper discs were sterilised by rinsing in ethanol. For statistical significance 3 replicate samples were seeded for each type of material, along with 3 unseeded (blank) materials. Cells were seeded at a concentration of 40,000 cells per well in a 48 well plate and cultured for 48 hours.

### Alamar Blue Assay

The Alamar blue assay (Serotec, UK) demonstrates the metabolic activity of cells by detection of mitochondrial activity. Cells incorporate the indicator dye that is reduced and excreted as a fluorescent product. Medium was removed from wells, cells rinsed in Earle's Balanced Salt Solution (EBSS) then 500µl of a 1:20 Alamar Blue:Hank's Balanced Salt Solution (HBSS) added to each well. Plates were incubated at 37°C for one hour, the solution removed to a fresh plate and 100µl of each solution read on cytofluor (PerSeptive Biosystems) at 535nm emission, 590nm absorbance. Blank values were extracted from experimental values to eliminate background readings.

### Statistics

Mean values and standard deviations (SD) were computed for three replicates per sample. The analysis of variance (ANOVA) was calculated along with Tukey-Kramer multiple comparison test to compare gamma-irradiated or non-irradiated samples of different molecular weights. Student's t-test was used to compare gamma irradiated and non-irradiated samples of the same molecular weights.

### Toluidine Blue Staining

Cells were rinsed several times in PBS, fixed in 1.5% gluteraldehyde in 0.1M phosphate buffer for 30 minutes, rinsed with PBS and stained with 1% Toluidine Blue in 0.05M phosphate buffer for 5 minutes. This solution was removed, cells were rinsed and covered with PBS and could then be photographed under the dissecting microscope.

### Scanning Electron Microscopy (SEM)

After toluidine blue staining, cells were fixed in osmium tetroxide for 30 minutes. Thereafter specimens were dehydrated through a series of ascending alcohols (50%-100%) dried in Hexamethyldisilazane (HMDS) and left to air dry before sputter coating with gold. Samples were then viewed in a Philips 501B SEM.

### Results

### Molecular Weight Distribution

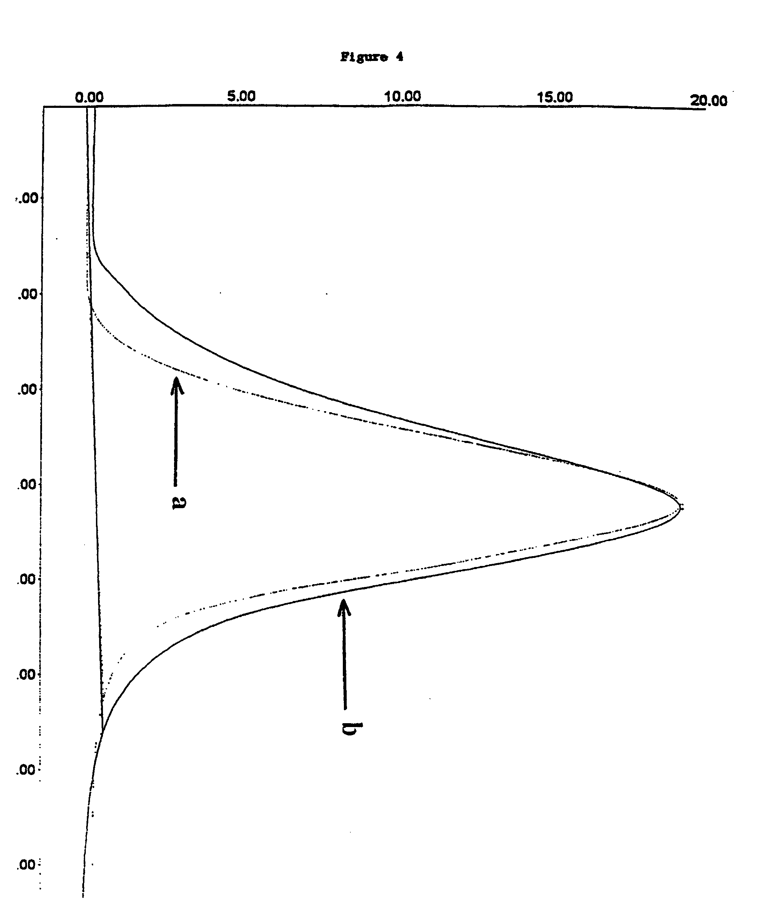

Table 2 gives the measured molecular weights and polydispersities of both the unsterilised and gamma sterilised samples. Significant differences exist between the theoretical Mn and the measured value however the PCL does show a range of molecular weights increasing in the correct order. Measuring definitive molecular weights of PCL is difficult due to the lack of a PCL standard for calibration. To obtain a more accurate figure would require the use of solution viscosity techniques. However the results do show some interesting trends, in particular the reduction in Mn and the increase in Mw giving a greater Pd for the gamma sterilised samples. Figure 4 shows a comparison of the GPC curves for the unsterilised and gamma sterilised PCL 75 highlighting the broadening of the peak due to the increase in low molecular weight material. Hence it is likely that the gamma radiation is breaking some of the longer polymer chains.

### Tensile Modulus

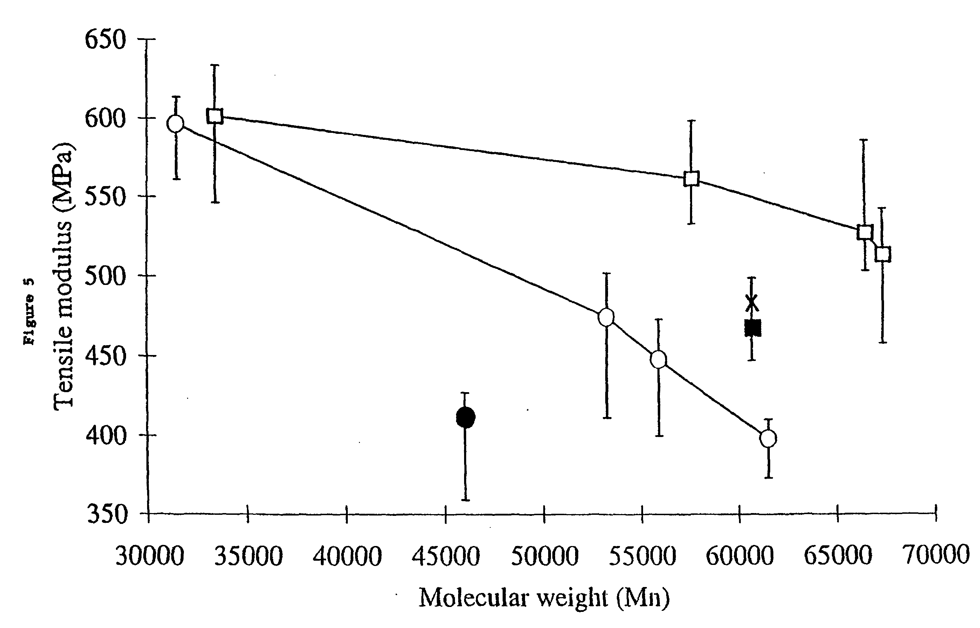

Figure 5 details the variation in tensile modulus of the PCL with molecular weight and the effects of gamma sterilisation upon the tensile modulus. Tensile modulus decreases with increasing molecular weight and there is a notable decrease in tensile modulus after gamma sterilisation. This is also the case for the CAPA 650 reference material which, interestingly, has a lower tensile modulus than the material produced using the *in-situ* polymerisation technique. The measured value of tensile modulus for the unsterilised CAPA 650 material is within 2% of the value given in the Solvay Interox literature ²².

### NMR and IR Spectra

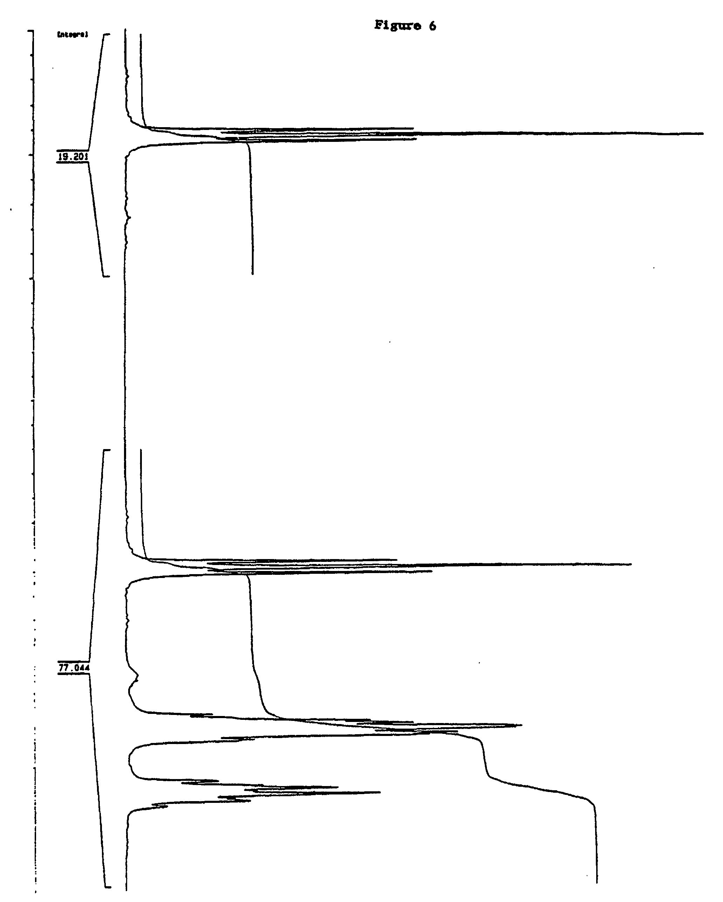

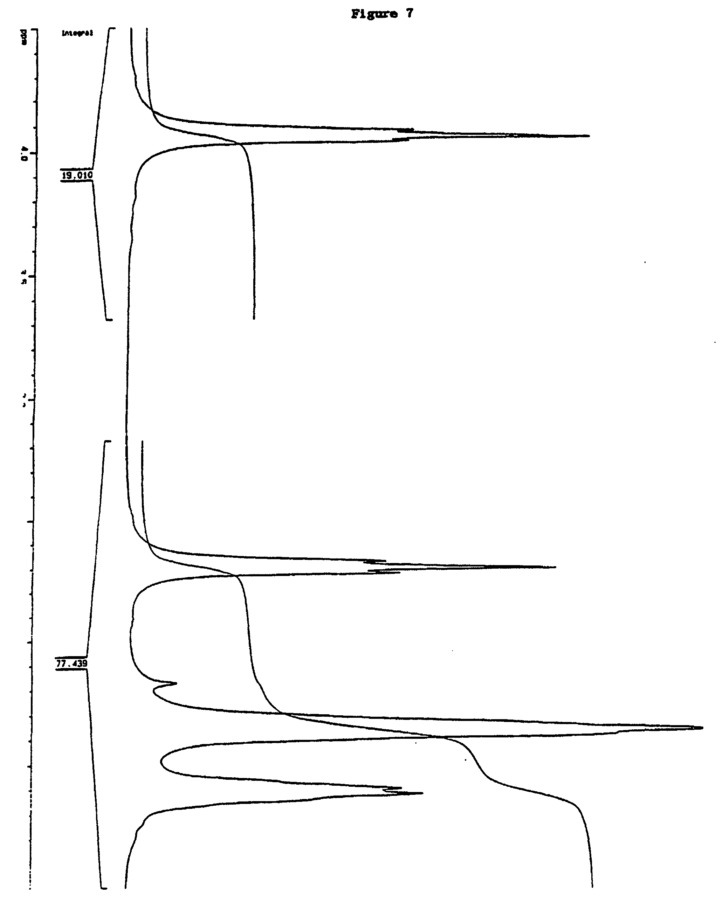

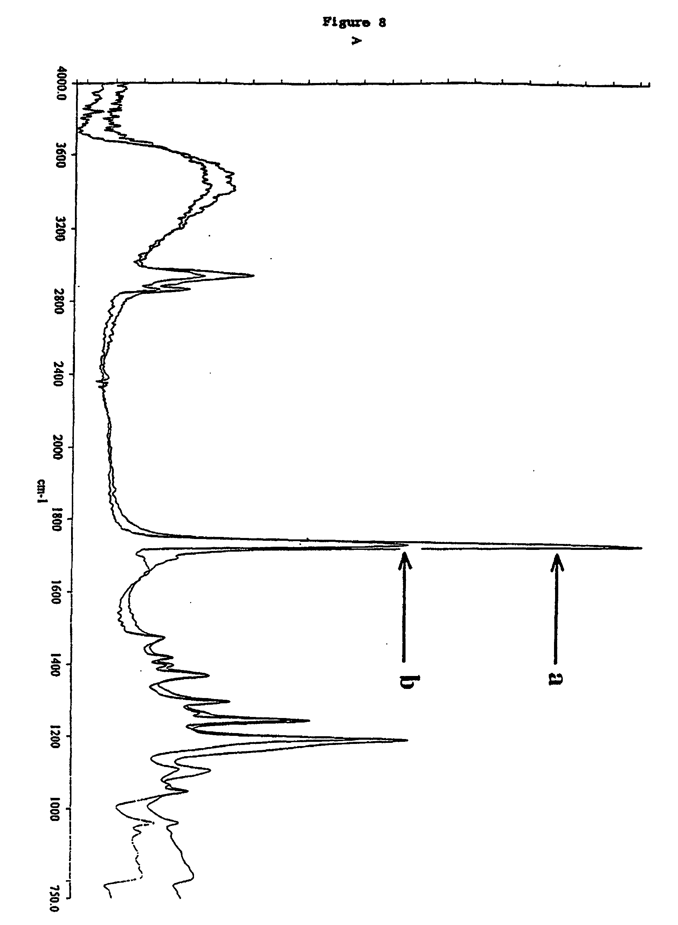

Figures 6, 7 and 8 shows the H¹ NMR spectra for both the PCL 50 and CAPA 650 material. The spectra show OCH₂ at 4.1; CH₂ - C = O at 2.3 and the hydrocarbon section at 1.3-1.8 ppm. The infra-red spectra show a carbonyl at ~ 1750 associated with the carbonyl in the backbone of the polymer. Both NMR and IR data agree with the samples of standard polymer indicating the material to be of the same type.

### DSC Results

Results from the DSC testing are given in table 3. The Tm and crystallinity values are within the range expected for PCL and agree with the data given by Solvay however repeating the tests for both unsterilised and gamma sterilised CAPA 650 material did not give highly repeatable results.

### PCL/Vicryl Composites

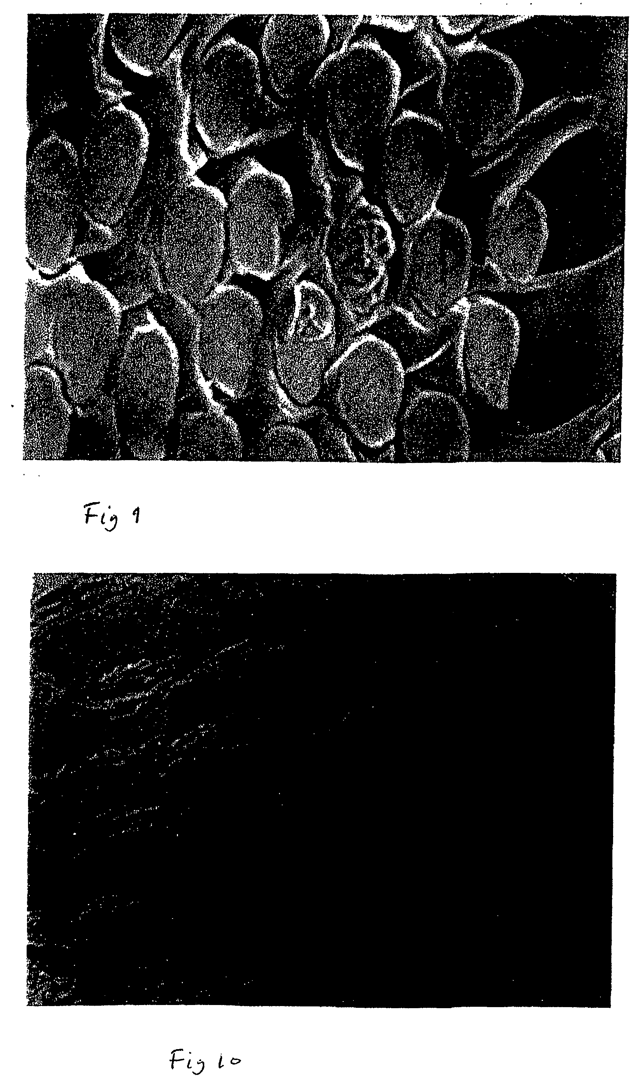

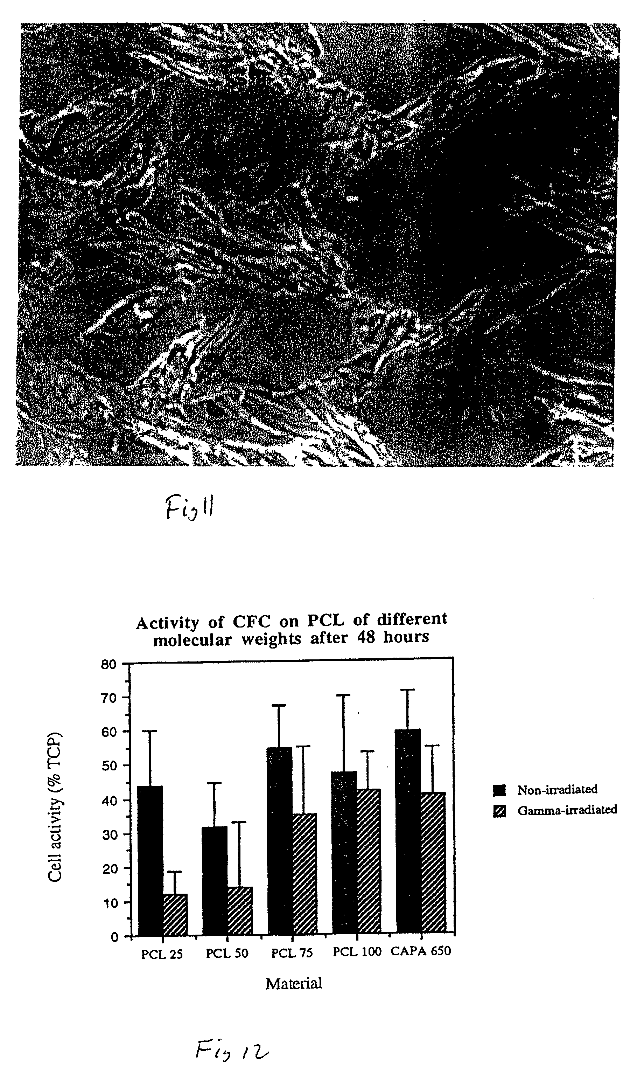

Figures 9, 10 and 11 are SEM micrographs of the composite materials. Clearly visible are the knitted and woven structures of the Vicryl mesh. Figure 11 shows a cross section of one of the yarns from the knitted composite material with the individual fibres fully encapsulated by PCL demonstrating the success of the technique for wetting out and infiltrating the fibre tows.

### Biocompatibility

Biocompatibility of CFC on PCL of different molecular weights, both gamma-irradiated and non-irradiated, was assessed by measuring cell activity and viewing cell morphology on the polymer surface after 48 hours incubation. On TCP cells attached and spread, forming a confluent layer after 48 hours. Cells were arranged in a swirling pattern, individual cells had a long, thin, spindly morphology. Cells attached and spread with good morphology on PCL of different molecular weights. On PCL 25-100 cells had a good morphology similar to that seen on TCP. There was complete cell coverage on 8mm diameter discs but there was not always a completed covering of cells on the surface of 10mm diameter discs. The topography of the surface was not always consistent and this may have some bearing on the cell attachment and spreading, and thus activity. If grooves were present on the surface, cells aligned along them. If there was a rough surface the cells did not attach. Holes were present on the surface of some of the polymer discs, cells appear to grow round them or span across them but did not grow into them. On CAPA 650, where the surface was very smooth with some holes in it, cells grew in stellar groups with an extremely flat morphology, much more so than on TCP or PCL 25-100. There was no cell attachment on the copper discs.

Cell activity was assessed by the Alamar Blue assay as shown in Figure 12. There was no significant difference in cell activity on non-irradiated PCL of different molecular weights and CAPA 650. All samples had significantly lower activity than TCP and significantly higher than copper and blank polymers, with the exception of PCL 50. Cell activity was not significantly different for gamma-irradiated PCL 75, 100 and CAPA 650, all lower than TCP and higher than copper or blank polymer. On gamma irradiated PCL 25 and 50 very few cells had attached to the surface and consequently, the cell activity was significantly lower than on PCL 75, 100 and CAPA 650 and not significantly different to copper or blank polymers. There was no significant difference between cell activity and gamma-irradiated and non-irradiated PCL 75, 100 and CAPA 650. Activity on gamma-irradiated PCL 25 and 50 was significantly lower than on non-irradiated PCL 25 and 50.

### Discussion

Initial investigations into the development of this novel *in-situ* polymerisation technique for PCL have produced excellent results. GPC, NMR and IR analysis has proved the material to have similar properties to a commercially available PCL material used as a benchmark.

Results from the tensile testing show the *in-situ* polymerised material to have a tensile modulus which is dependant upon molecular weight. The tensile modulus decreases with molecular weight. In all cases except for the gamma sterilised PCL 100 material the *in-situ* polymerised PCL had a higher tensile modulus than the gamma sterilised CAPA 650 indicating that with our novel manufacturing technique we can obtain a tensile modulus greater than or comparable to our benchmark material.

Results from the IR and NMR analysis indicate that the *in-situ* polymerised material is of a similar chemical composition to the CAPA 650 material. GPC analysis has indicated that we can obtain similar or greater molecular weights to our benchmark material with particularly narrow molecular weight distributions.

Biocompatibility results show that the CFC cells will attach and spread on PCL of different molecular weights, although this depends somewhat on the surface topography of the discs. Different surface topographies were due to the different machined finish on the upper and lower PTFE mould surfaces and the ground surface finish used on the compression moulding platens for the CAPA 650 material. There was no difference in cell activity on gamma-irradiated or non-irradiated polymers with the exception of PCL 25 and 50.

Using this *in-situ* polymerisation technique as a variant of RTM to produce totally absorbable long fibre composite materials has produced encouraging results. The Vicryl fibres appear to be well wet out and encapsulated within the PCL matrix giving a two phase material. Due to their low modulus the Vicryl fibres are having little reinforcing effect, however, the use of a higher modulus bioabsorbable glass fibre will allow us to control the material mechanical properties. The use of such a low pressure liquid moulding technique should allow the fabrication of patient specific implants made from low cost tooling produced directly from CT scan data using rapid prototyping techniques.

### Conclusions

A novel manufacturing process for PCL has been developed based upon RTM, an established technique for producing composite materials using thermosetting matrices. Preliminary comparisons of the physical and biocompatibility properties of the PCL material produced using this *in-situ* polymerisation approach compared with a commercially available PCL material (CAPA 650) have produced encouraging results. NMR and IR analysis show that the chemical composition of the *in-situ* polymerised material is that of PCL. GPC analysis has demonstrated that the material can be produced with a variable molecular weight and a narrow molecular weight distribution. Tensile testing results indicate a slightly higher tensile modulus for the *in-situ* polymerised material compared to the CAPA 650. The effect of sterilisation by gamma irradiation has been investigated producing a broader molecular weight distribution and slight reduction in tensile modulus.

In-vitro biocompatibility of both the *in-situ* polymerised PCL and CAPA 650 material has been assessed using osteoblasts derived from human craniofacial bone cells. The material is highly biocompatible with these cells which will attach and spread on both the irradiated and non-irradiated PCL and CAPA 650. The main factor influencing cell behaviour seems to be the surface topography of the polymer samples.

Long fibre composite materials have been produced using both woven and knitted Vicryl meshes. SEM micrographs show the fibre to be fully wet-out and encapsulated by the PCL matrix material.

### REFERENCES

Rudd, C. D., Kendall, K.N., Long, A. C., Mangin, C.E. Liquid moulding technologies. Woodhead Publishing 1997.

2. Biodegradable CAPA Thermoplastics. CAPA 650 data sheet. Solvay Interox.

## Claims

1. Biodegradable fibre-reinforced shaped composite suitable for use as a medical implant comprising matrix and fibres wherein the matrix and fibres display differential rates of biodegradation as a function of the nature of material or molecular weight thereof **characterised in that** the shaped composite comprises a shaped fibre preform of predetermined fibre distribution, orientation and/or fraction and the matrix is selected from polymers and co-polymers of aliphatic polyesters, preferably poly-ε-caprolactone such that in use the matrix and/or fibre biodegrade via an intermediate comprising residual porous matrix or residual fibre form respectively providing voids suitable for primary growth of cells or providing a residual scaffold for attachment and growth of cells.

2. Biodegradable fibre-reinforced shaped composite according to Claim 1 wherein the matrix and fibres comprise a combination of materials whereby a differential degradation rate is exhibited both within and between the matrix and/or fibre.

3. Biodegradable fibre-reinforced shaped composite according to Claim 1 or 2 wherein the fibre reinforcement is selected from ceramics such as beta-tricalcium phosphate and phosphate free calcium aluminium (Ca-Al), bioglasses such as the glass form of calcium phosphate, calcium metaphosphate (CMP) and calcium sodium metaphosphate (CSM), mixtures of silica, sodium oxide, calcium oxide and phosphorus pentoxide, and polymeric materials as defined in Claim 1.

4. Biodegradable fibre-reinforced shaped composite as defined in any of Claims 1 to 3 which is coated with or associated with or has embedded therein or is impregnated with a selected population of host and/or compatible donor cells, preferably bone derived and/or cartilage derived and/or collagen derived.

5. Biodegradable composite according to Claim 4 comprising primary growth cells selected from bone, cartilage and tissue cells suitable for providing a supporting structure of live bone or cartilage or a live vascular structure within the partially biodegraded composite, adapted for further growth of remaining cell types for total integration as a functioning live system.

6. Biodegradable composite according to any of Claims 4 and 5 suitable as a surgical implant for reconstruction of bone or cartilage or of soft tissue, muscle **characterised by** primary degradation rate of the matrix or of the fibre respectively.

7. Process for producing a biodegradable fibre-reinforced shaped composite as hereinbefore defined in any of Claims 1 to 3 comprising providing the said shaped fibre preform comprising a presentation of reinforcing fibres in a regular, irregular or profiled fibre distribution in a tool or mould;
injecting into said preform within said tool or mould a composition comprising monomers or co monomers and/or oligomers and/or resin of the said polymer matrix in such a manner as to retain said distribution, orientation and/or fraction, of fibres and composite shape; and
polymerising or part polymerising the composition in the mould or tool.

8. Process as claimed in Claim 7 for the production of a shaped product, wherein the mould or tool comprises a 3-dimensional template of a 3-dimensional image of a selected feature or area of a patient for implant.

9. Process according to Claim 8 wherein the mould or tool is provided by
(i) medical imaging of a selected feature or area of a patient complementary to or symmetrical with a feature or area to be replaced and/or restructured to obtain data comprising a plurality of coordinates defining a three dimensional image;
(ii) passing data collected from medical imaging to a translating system which interprets said data and generates information for transferring said data to a rapid prototyping system suitable for generating a mould or tool;
and wherein fibre comprises long continuous fibres which are 10² -10⁴ times greater in length than diameter providing directional reinforcement.

## Patentansprüche

1. Biodegradierbares, faserverstärktes geformtes Komposit, das zur Verwendung als medizinisches Implantat geeignet ist, umfassend eine Matrix und Fasern, wobei die Matrix und die Fasern abweichende Biodegradierungsraten als Funktion der Natur des Materials oder seiner Molekularmasse zeigen, **dadurch gekennzeichnet, dass** das geformte Komposit eine geformte Faservorform vorgegebener Faserverteilung, -orientierung und/oder -teilung umfasst und die Matrix ausgewählt ist aus Polymeren und Kopolymeren aliphatischer Polyester, vorzugsweise Poly-ε-Caprolactonen, so dass bei Verwendung die Matrix und/oder Fasern über ein Zwischenprodukt biodegradieren, dass Reste poröser Matrix bzw. Restfaserform umfasst, die zum primären Wachstum von Zellen geeignete Lücken bereitstellen oder ein Restgerüst zum Anlagern oder Wachsen von Zellen bereitstellen.

2. Biodegradierbares, faserverstärktes geformtes Komposit gemäß Anspruch 1, wobei die Matrix und die Fasern eine Kombination von Materialien umfassen, wobei sich eine abweichende Abbaurate sowohl in als auch zwischen der Matrix und/oder der Faser zeigt.

3. Biodegradierbares, faserverstärktes geformtes Komposit gemäß Anspruch 1 oder 2, wobei die Faserverstärkung ausgewählt ist aus Keramik wie etwa Beta-Tricalciumphosphat und Phosphat-freiem Calcium-Aluminium (Ca-Al), Biogläsern wie etwa der Glasform von Calciumphosphat, Calciummetaphosphat (CMP) und Calciumnatriummetaphopshat (CSM), Mischungen von Kieselerde, Natriumoxid, Calciumoxid und Phosphorpentoxid, und polymeren Materalien wie in Anspruch 1 definiert.

4. Biodegradierbares, faserverstärktes geformtes Komposit gemäß einem der Ansprüche 1 bis 3, das mit einer ausgewählten Population von Wirts- und/oder kompatiblen Spenderzellen, vorzugsweise aus Knochen erhalten und/oder aus Knorpel erhalten und/oder aus Kollagen erhalten, beschichtet oder assoziiert ist oder diese einbettet oder mit ihnen imprägniert ist.

5. Biodegradierbares Komposit gemäß Anspruch 4, umfassend primäre Wachstumszellen, die ausgewählt sind aus Knochen, Knorpel und Gewebezellen, die zum Bereitstellen einer Trägerstruktur lebenden Knochens oder Knorpels oder einer lebenden vaskulären Struktur innerhalb des teilweise biodegradierbaren Komposits geeignet sind, ausgelegt für das weitere Wachstum der verbleibenden Zellarten für eine vollständige Integration als ein funktionierendes lebendes System.

6. Biodegradierbares Komposit gemäß einem der Ansprüche 4 und 5, geeignet als ein chirurgisches Implantat zur Rekonstruktion von Knochen oder Knorpel oder von weichem Gewebe, Muskel, **gekennzeichnet durch** eine primäre Degradierungsrate der Matrix bzw. der Faser.

7. Verfahren zum Herstellen eines biodegradierbaren faserverstärkten geformten Komposits, wie vorstehend in einem der Ansprüche 1 bis 3 definiert, umfassend Bereitstellen der geformten Faservorform, die eine Darbietung verstärkender Fäsern in einer gleichmäßigen, ungleichmäßigen oder fassonierten Faserverteilung in einem Werkzeug oder einer Form umfasst;
Einspritzen einer Zusammensetzung, die Monomere oder Comonomere und/oder Oligomere und/oder Polymer der Polymermatrix in die Vorform im Werkzeug oder in der Form in einer solchen Weise umfasst, dass die Verteilung, Orientierung und/oder Teilung von Fasern und die Kompositform erhalten bleiben; und Polymerisieren oder teilweise Polymerisieren der Zusammensetzung in der Form oder dem Werkzeug.

8. Verfahren gemäß Anspruch 7 zur Herstellung eines geformten Produkts, wobei die Form oder das Werkzeug eine dreidimensionale Schablone eines dreidimensionalen Bilds eines ausgewählten Merkmals oder Bereichs eines Patienten für ein Implantat umfasst.

9. Verfahren gemäß Anspruch 8, wobei die Form oder das Werkzeug durch
(i) Medizinische Bildgebung eines ausgewählten Merkmals oder Bereichs eines Patienten komplementär oder symmetrisch zu einem zu ersetzenden und/oder zu restrukturierenden Merkmal oder Bereich, um Daten zu erhalten, die eine Mehrzahl von ein dreidimensionales Bild definierenden Koordinaten umfassen;
(ii) Weitergeben von aus medizinischer Bildgebung gewonnenen Daten an ein Übersetzungssystem, das die Daten interpretiert und Informationen zum Übertragen der Daten zu einem Schnellprototyp-System erzeugt, das zum Erzeugen einer Form oder eines Werkzeugs geeignet ist;
bereitgestellt wird und wobei die Fasern lange, kontinuierliche Fasern umfassen, die in der Länge 10²-10⁴ fach größer sind als im Durchmesser und gerichtete Verstärkung bereitstellen.

## Revendications

1. Composite mis en forme renforcé par des fibres biodégradable adapté pour une utilisation en tant qu'implant médical comprenant une matrice et des fibres, dans lequel la matrice et les fibres présentent des vitesses de biodégradation différentielles en fonction de la nature du matériau ou de la masse moléculaire de celles-ci, **caractérisé en ce que** le composite mis en forme comprend une préforme de fibres mise en forme de répartition, de disposition et/ou de fractionnement des fibres prédéterminés et la matrice est choisie parmi les polymères et les copolymères de polyesters aliphatiques, de préférence le poly-ε-caprolactone, de sorte qu'en utilisation la matrice et/ou les fibres se biodégradent via un produit intermédiaire comprenant une forme de matrice poreuse résiduelle ou de fibres résiduelles fournissant respectivement des vides adaptés pour une croissance primaire de cellules ou fournissant une base résiduelle pour la fixation ou la croissance de cellules.

2. Composite mis en forme renforcé par des fibres biodégradable selon la revendication 1, dans lequel la matrice et les fibres comprennent une combinaison de matériaux, moyennant quoi une vitesse de dégradation différentielle est présente à la fois à l'intérieur et entre la matrice et/ou les fibres.

3. Composite mis en forme renforcé par des fibres biodégradable selon la revendication 1 ou la revendication 2, dans lequel le renforcement par des fibres est choisi parmi les céramiques telles que le bêta-phosphate tricalcique et le calcium-aluminium sans phosphate (Ca-Al), le bioverre tel que la forme vitreuse du phosphate de calcium, du métaphosphate de calcium (MPC) et du métaphosphate de sodium et de calcium (MSC), les mélanges de silice, d'oxyde de sodium, d'oxyde de calcium et de pentoxyde de phosphore, et les matériaux polymérisés tels que définis dans la revendication 1.

4. Composite mis en forme renforcé par des fibres biodégradable selon l'une quelconque des revendications 1 à 3, qui est revêtu de ou associé à ou est incrusté du ou est imprégné d'une population sélectionnée de cellules hôtes et/ou de cellules donneuses compatibles, de préférence dérivées de l'os et/ou dérivées du cartilage et/ou dérivées du collagène.

5. Composite biodégradable selon la revendication 4 comprenant des cellules de croissance primaire sélectionnées à partir de cellules d'os, de cartilage et de tissu adaptées pour fournir une structure de support de cartilage ou d'os vivant ou une structure vasculaire vivante à l'intérieur du composite partiellement biodégradé, adapté pour une croissance supplémentaire des types de cellules restantes pour une intégration totale en tant que système vivant en fonctionnement.

6. Composite biodégradable selon l'une quelconque des revendications 4 et 5 adapté en tant qu'implant chirurgical pour une reconstruction d'os ou de cartilage ou de tissus mous, **caractérisé** au niveau musculaire par une vitesse de dégradation primaire de la matrice ou des fibres respectivement.

7. Méthode pour produire un composite mis en forme renforcé par des fibres biodégradable selon l'une quelconque des revendications 1 à 3 comprenant la fourniture de ladite préforme de fibre mise en forme comprenant une présentation de fibres de renforcement selon une répartition des fibres régulière, irrégulière ou profilée dans un outil ou un moule ;
l'injection dans ladite préforme à l'intérieur dudit outil ou moule d'une composition comprenant des monomères ou des comonomères et/ou des oligomères et/ou de la résine synthétique de ladite matrice polymère de manière à retenir ladite répartition, ladite orientation et/ou ledit fractionnement des fibres et de la forme du composite ; et
la polymérisation entière ou partielle de la composition dans le moule ou l'outil.

8. Méthode selon la revendication 7 pour la production d'un produit mis en forme, dans laquelle le moule ou l'outil comprend un gabarit tridimensionnel d'une image tridimensionnelle d'une caractéristique ou zone sélectionnée d'un patient pour un implant.

9. Méthode selon la revendication 8, dans laquelle le moule ou l'outil est fourni
(i) par imagerie médicale d'une caractéristique ou d'une zone sélectionnée d'un patient complémentaire de ou symétrique à une caractéristique ou une zone à remplacer et/ou restructurer pour obtenir des données comprenant une pluralité de coordonnées définissant une image tridimensionnelle ;
(ii) par la transmission des données recueillies à partir de l'imagerie médicale à un système de traduction qui interprète lesdites données et génère des informations pour transférer lesdites données à un système de prototypage rapide adapté pour générer un moule ou un outil ;
et dans laquelle les fibres comprennent de longues fibres continues qui sont 10² à 10⁴ fois plus grandes en longueur par rapport au diamètre fournissant un renforcement directionnel.
